Europäisches Patentamt

European Patent Office

Office européen des brevets

(11) Publication number: **0 163 502**
**B1**

(12) **EUROPEAN PATENT SPECIFICATION**

(45) Date of publication of patent specification: **18.07.90**

(21) Application number: **85303693.7**

(22) Date of filing: **24.05.85**

(60) Divisional application **89202513.1** filed on **24/05/85.**

(51) Int. Cl.⁵: **A 61 B 17/22, A 61 B 17/32, A 61 M 29/02, A 61 M 25/00**

(54) Atherectomy device.

(30) Priority: **30.05.84 US 615298**
**10.05.85 US 732691**

(43) Date of publication of application:
**04.12.85 Bulletin 85/49**

(45) Publication of the grant of the patent:
**18.07.90 Bulletin 90/29**

(84) Designated Contracting States:
**AT BE CH DE FR GB IT LI LU NL SE**

(56) References cited:
**EP-A-0 086 048**
**WO-A-82/00408**
**FR-A-1 161 400**
**GB-A-2 044 103**
**US-A-3 811 448**
**US-A-3 990 453**
**US-A-4 295 464**

(73) Proprietor: **DEVICES FOR VASCULAR INTERVENTION INC.**
**750 Welch Road**
**Palo Alto California 94304 (US)**

(72) Inventor: **Simpson, John Bush**
**116 Fox Hollow Road**
**Woodside California 94062 (US)**

(74) Representative: **Bayliss, Geoffrey Cyril et al**
**BOULT, WADE & TENNANT 27 Furnival Street**
**London EC4A 1PQ (GB)**

Courier Press, Leamington Spa, England.

## Description

This invention relates to an atherectomy device for removing or minimizing atheromas and for taking biopsies.

Peripheral vascular arteriosclerosis is a common ailment occuring in humans which involves the deposition of a fatty-like substance called atheromas or plaque in blood vessels and particularly in the peripheral blood vessels that feed the limbs of the human body. Occasionally these fatty deposits occur in fairly localised regions in the blood vessel. Substantial success has been obtained in increasing the size of the flow passages in the arteries in which these deposits have occurred by the use of a dilitation process using balloon angioplasty. However, it has been found that in a substantial percentage as, for example, 20 to 30% of the cases which are treated in this manner there is a tendency for the atheromas to reoccur. There is a need for a device which will substantially reduce such reoccurrances and thereby eliminate the need for additional dilitations.

FR—A—1161400 discloses an automatic instrument for performing biopsies comprising a cylinder formed with a side aperture and secured to a flexible sheath. The cylinder is provided with an annular cutter to which rotational and translation movement is imparted by a flexible tube connected thereto and which slides inside the flexible sheath. In use the apertured cylinder is placed under vacuum.

This invention provides an atherectomy device for removal of material of an atheroma in a vessel comprising a generally cylindrical housing formed of a rigid material, said housing having a rounded distal end portion, said housing being formed with a cut-out on one side of the housing, cutter means disposed within the housing and movable in the housing longitudinally of the cut-out, a flexible guiding means secured to the housing, a flexible drive means disposed within the guiding means and secured to the cutter, and motive means secured to the drive means for rotating the cutter and permitting rotation of the cutter while the cutter is moved longitudinally of the housing; wherein the cut-out extends longitudinally of the housing so that the remaining portion of the housing in the vicinity of the cut-out forms substantially a semi-cylinder.

The device may further comprise an inflatable means carried by the housing in a region opposite the cut-out, a tube connected to the inflatable means and having a lumen therein in communication with the interior of the inflatable means and means for inflating the inflatable means so that the housing can be urged in a direction towards the atheroma to retain the housing in engagement with the atheroma during the operation of the cutter means.

More specifically an additional tube may enclose said tube in communication with the inflatable means and guiding means.

An adapter may be secured to the guiding means and means may be for introducing a radiographic contrast liquid into the adapter and into the torque tube.

Said motive means may include an electric motor having an output shaft and clutch means for connecting the output shaft of the electrical motor to the flexible drive means.

In any of the above arrangements the device may comprise first and second cylindrical members, the second cylindrical member being slidably mounted in the first cylindrical member, a motor carried by the second movable member, clutch means connecting the output shaft of the motor to the torque cable and means carried by the first and second cylindrical members permitting the first and second cylindrical members to be held by a single hand and permitting longitudinal movement of the second cylindrical member with respect to the first movable member. The arrangement may further comprise switch means carried by the second cylindrical member whereby the motor can be operated by the same hand that is holding the first and second cylindrical members.

Means may be carried by the second movable member for causing engagement and disengagement of the clutch means and means carried by the first and second cylindrical members to prevent accidental disengagement of the clutch means during the time that the cutter is being moved longitudinally of the housing.

The following is a specific description of a number of preferred embodiments of the invention, reference being made to the accompanying drawings in which:

Figures 1A and 1B are side elevational views with certain portions broken away of an atherectomy device incorporating the present invention in which Figure 1A typically is approximately on a 10 to 1 scale and Figures 1B, except for the inflating device, typically is approximately on a 1 to 2 scale;

Figures 2A through 2E show how the atherectomy device shown in Figure 1 is utilised;

Figure 3 is a partial side elevational view with certain portions broken away of another embodiment of the atherectomy device incorporating the present invention;

Figure 4 is a cross section view taken along the line of Figure 3;

Figure 5 is a cross section view taken along the line 5—5 of Figure 3;

Figures 6A and 6B show another embodiment of an atherectomy device incorporating the present invention in which the advancement and rotation of the cutter is motorized;

Figure 7 is an enlarged detail view of the distal extremity of the atherectomy device shown in Figure 6A and 6B; and

Figure 8 is an enlarged cross sectional view taken along the line 8—8 of Figure 6A.

In general, the atherectomy device is comprised of a generally cylindrical housing formed of a relatively rigid material. The housing has rounded distal and proximal end portions. The housing is formed with a cutout extending longitudinally of the housing. Atheroma cutting means is disposed within the housing. Flexible guide means is secured to the proximal end of the housing for advancing the housing into the arterial vessel. Flexible drive means extends through the flexible guide means and is connected to the atheroma cutting means in the housing for causing operation of the atheroma cutting means to remove at least a portion of an atheroma from the arterial vessel.

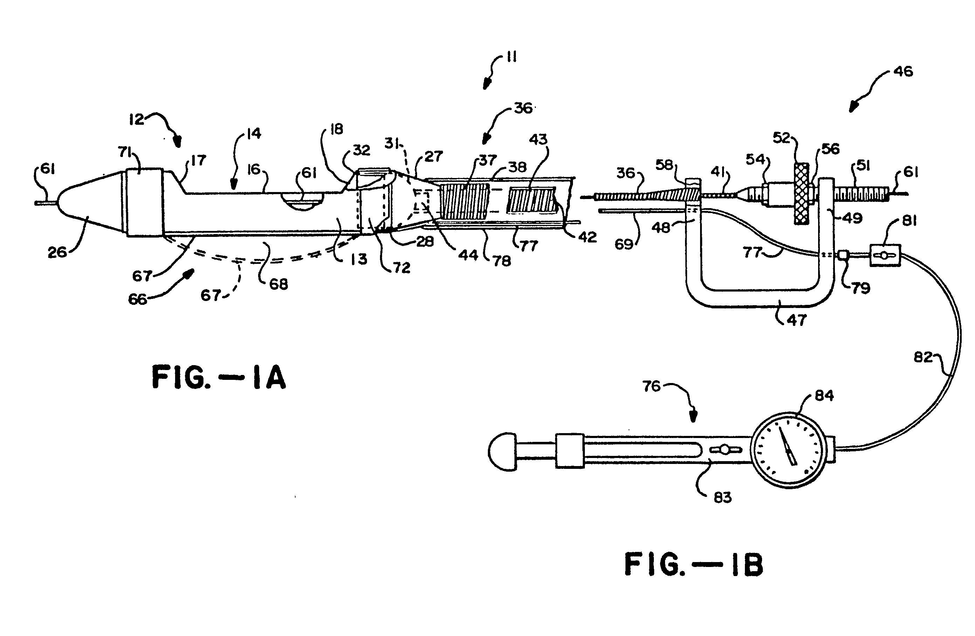

More particularly the atherectomy device 11 as shown in Figures 1A and 1B of the drawings consists of a housing 12 which has a generally cylindrical configuration. The housing 12 can be formed of any suitable relatively rigid materials as, for example, stainless steel. However, if desired a rigid plastic can be utilized, particularly if the device is to be disposable. The housing 12 consists of a cylinder 13 having a suitable diameter as, for example, ranging from 0.0762 cms to 0.381 cms (.030" to .150"). The cylinder 13 is open-ended and is provided with a cutout 14 extending longitudinally of the same. The cutout 14 is formed so that the remaining portion of the cylinder in the vicinity of the cutout forms substantially a semi-cylinder. The cutout is defined by planar surfaces 17 of the sidewall of the cylinder lying in a plane parallel to the longitudinal axis of the cylinder 13 and upwardly and outwardly inclined end surfaces 17 and 18 adjoining opposite ends of the surfaces 17.

The housing 12 is provided with rounded end portions to facilitate movement of the housing within the coronary arteries. Thus on the distal extremity of the housing 12, there is provided a generally conical member 26 which is provided with a rounded extremity. The conical member 26 also is formed of a suitable material such as stainless steel or plastic. As shown the member 26 can be formed integral with the housing 12. Alternatively, it can be secured to the cylinder 13 by suitable means such as laser welding or a suitable adhesive.

A separate conical member 27 is secured to the proximal end of the cylinder 13. The proximal end of the cylinder 13 is provided with an annular recess 28 which is adapted to receive the conical member 27 and secured to the cylinder 13 by suitable means such as laser welding or by an adhesive. The housing 12 when so constructed has a smooth outer surface. The conical member 27 is truncated as shown.

Atheroma cutting means 31 is disposed within the housing 12. The cutting means 31 is cup-shaped as shown and is sized in such a manner so that it can fit into the cylinder 13 of the housing 12 and can be slidably moved longitudinally of the housing as hereinafter described. The cup-shaped cutting member 31 is provided with a circular continuous cutting edge 32 which lies in a plane perpendicular to the longitudinal axis of the housing 12. However, it should be appreciated that if desired the cutting edge can have other configurations. For example, the cutting edge can be inclined to form an elipse. Also the cutting edge can be undulating if desired. The atheroma cutting means is inserted into the housing 12 before the conical member 27 is secured to the cylinder 13.

Flexible guiding means 36 is secured to the proximal extremity of the housing 12. The flexible guiding means 36 is in the form of an elongate hollow cylindrical cable-like member or coil spring 37 formed of a suitable material such as stainless steel. The cable-like member or spring 37 is covered with a tubular member 38 of suitable material such as a heat shrinkable plastic to prevent the coils of the cable-like member or spring from separating and also to enhance the type of control which can be provided by the cable-like member or spring so that the housing 12 and the flexible guiding means 36 can follow different curves in the blood vessels and tortuous segments of blood vessels. The flexible guiding means 36 can be secured to the truncated end of the conical member 27 by suitable means such as welding or an adhesive.

Flexible drive means 41 is provided for operating the cutting means 31 and is also formed of an elongate cylindrical cable-like member or coil spring 42 of a suitable material such as stainless steel and in which the cable-like member or spring 42 is covered with a tubular member 43 of suitable material such as a heat shrinkable plastic to prevent separation of the coils of the spring or cable 42 and also to facilitate movement of the flexible drive means 41 through the flexible guiding means 36. The flexible drive means 41 is secured to the proximal extremity of the atheroma cutting means 31 as shown particularly in Figure 1 by welding or brazing the spring onto a stud 44 forming a part of the cutting means 31. In certain applications it may be desirable to use a plastic tube formed of a suitable plastic such as Teflon (Registered Trade Mark) in place of the cable-like members used in the flexible guiding means 36 the flexible drive means 41. Such a tube has been found to supply the necessary torsional strength.

Control means 46 is provided for controlling the movement of the housing 12 and operation of the cutting means 31 and for providing relative rotational movement between the same and movement of the cutting means 31 longitudinally of the axis of the housing 12. Such control means consists of a U-shaped control handle 47. The control handle is provided with spaced parallel legs 48 and 49. A threaded rod 51 is threaded into the leg 49 and extends in a direction at right angles to the leg 49. A knurled thumb screw 52 is rotatably mounted on the threaded rod 51 and is movable longitudinally of the rod and can be locked in a predetermined position by two lock nuts 54 and 56 adjustably positioned on the rod 51. The proximal extremity of the flexible guiding means 36 is mounted in a hole 58 provided in the leg 48 and is secured therein by a suitable means such as an adhesive. The flexible drive means 41 extends through the flexible guiding means 36 and has its proximal extremity secured to the tapered forward extremity of the threaded rod 51 by suitable means such as an epoxy. The knurled knob is positioned so that when the lock nut 56 is adjacent the leg 49, the cutting means 31 is in its rearmost extremity and when the lock nut 54 is in a position engaging the other leg 48, the cutting means 31 is in its forwardmost position. This ensures that the spring which serves as the flexible drive means 41 is not stressed unduly.

A guide wire of conventional construction 61 is provided which extends through a bore (not shown) provided in the threaded rod 51 and through the interior of the flexible drive means 41 and thence through the cutting means 31, through the housing 12 and through the conical member 26 of the housing 12.

Inflatable means 66 is provided for moving the housing 12 after it has been advanced into the desired position in the arterial vessel into close proximity to the atheroma on which a cutting operation is to be performed. The inflatable means 66 is carried by the housing 12 exterior of the housing 12 in a region opposite the cutout 14. This inflatable means 66 takes the form of a balloon 67 formed in a conventional manner integral with and at the end of an elongate tubular member 69 formed of plastic. The balloon can be very thin walled and need only take pressures typically in a range from $1.37 \times 10^5$ to $2.055 \times 10^5$ N/m$^2$ (20 to 30 psi). The balloon 67 preferably extends the length of the cylinder 13 and is secured to the exterior surface of the housing 12 at opposite ends of the cylinder 13 by suitable means such as bands 71 and 72 formed of a heat shrinkable plastic.

Inflating means 76 is provided for supplying a fluid to the balloon 67 and includes the flexible tubular member 77 which provides a lumen which opens into the balloon 67. The member 77 extends along the flexible guiding means 36 to the control handle 47. It can be held in close proximity to the guiding means 36 by another heat shrinkable tubular member 78. The tubular member 77 is provided with a suitable fitting as, for example, a Luer adapter 79 which is adapted to be connected to suitable valve means as, for example, a two-way stop cock 81. The stop cock 81 is connected by a tube 82 to a balloon inflating device 83 called an "Indeflator" and described in United States Letters Patent No. 4,439,185. The Indeflator is used for introducing a fluid into the tubular member and to inflate the space 68 in the balloon 67 so that its outer side wall assumes the position represented. The pressure applied to inflate the balloon is observed by reading the gauge 84.

It should be appreciated that the tubular member 77, if desired, can be positioned within the flexible guiding means 36. Also, the U-shaped control handle 47 can be provided with openings or holes (not shown) in the arms 48 and 49 which can receive the tubular member 77.

Operation of the atherectomy device shown in Figures 1B and 1B may now be briefly described in conjunction with the method of the present invention as shown in Figures 2A to 2E. Let it be assumed that it is desired to cut out at least a portion of an atheroma in an arterial vessel. The arterial vessel 91 shown in Figure 2A has a wall 92 which has an atheroma 93 formed thereon. Let it also be assumed that it is desired to cut out at least a portion of the atheroma 93 in the arterial vessel 91. To accomplish this, an incision is made in the human body to make it possible to enter the arterial vessel in which the atheroma 93 is located. The distal tip of the guide wire 61 is inserted into the arterial vessel and advanced in the vessel until it extends through the arterial vessel through the stenosis, or narrowing 96 of the vessel created by the atheroma as shown in Figure 2A. Thereafter the proximal end of the guide wire 61 is threaded through atherectomy device 11 by inserting the guide wire 61 through the housing 12, the guiding means 36 and the threaded rod 51.

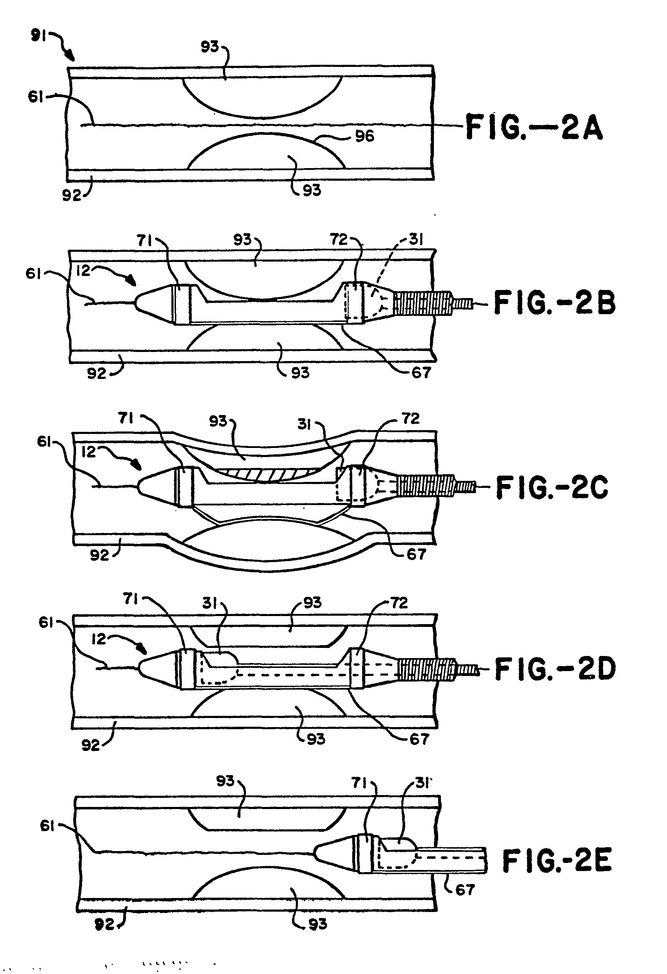

At this point in the operation, the cutting means 31 is completely retracted in the housing 12 to the position shown in Figure 1A of the drawings. The atherectomy device 11 is then inserted into the arterial vessel with the housing 12 going first. The flexible guiding means 36 provided for the housing 12 makes it possible for the atherectomy device to enter readily the arterial vessel and to follow the lumen in the arterial vessel. To facilitate advancement of the housing 12, the control handle 47 can be rotated back and forth. Advancement is continued until the housing 12 is positioned in the vicinity of the atheroma 93 as shown in Figure 2B. The positioning of the housing 12 can be observed by the use of a fluoroscope. The housing 12 as hereinbefore explained can be made of a material such as stainless steel which is relatively opaque to X-rays and thus gives a good image as the device is being positioned in the arterial vessel.

After the housing 12 is properly positioned as shown in Figure 2B, the balloon 67 is inflated by use of the inflating means 76 to force the housing 12 firmly into engagement with the atheroma 93 and particularly so that a substantial portion of the atheroma is positioned in the cutout 14. The inflation is accomplished by opening the stop cock 81 and introducing a fluid by operation of the Indeflator 83. The pressure at which the fluid is introduced into the balloon can be readily observed by reading the gauge 84 of the Indeflator 83. The balloon can be inflated with a saline solution or even air if desired since only a relatively low pressure is required, as for example, $1.37 \times 10^5$ to $2.055 \times 105$ N/w$^2$ (20 to 30 psi). The inflation pressure is substantially less than that used for dilitation because the purpose is merely to position the housing 12 so it is in a snug position against the atheroma.

The balloon 67 when inflated engages the opposite side of the atheroma received by the cutout 14. As seen, the balloon 67 when inflated can accommodate the configuration of the atheroma 93 and thus apply a desired equalizing pressure to urge the housing towards the atheroma in the manner shown in Figure 2C. As this is occurring it has been found that the vessel wall 92 bulges outwardly below the balloon and bulges inwardly on the opposite side so that the atheroma is depressed into the cutout 14 of the housing 12 as shown in Figure 2C.

The cutting means 11 may now be operated. This is accomplished by rotating the thumb screw 52. As the thumb screw 52 is rotated as, for example, in a clockwise direction, the threaded rod 51 upon which the thumb screw 52 is mounted is rotated in the arm 47 so that the thumb screw 52 is advanced. This movement of thumb screw 52 causes rotation and advancement of the flexible drive means 41 which causes rotation and advancement of the cutting means 31. It should be appreciated that either or both the flexible guiding means 36 or the flexible drive means 41 can be rotated in opposite directions to cause this rotation with longitudinal advancement. The inflated balloon 67 serves to maintain the housing in engagement with the atheroma 93 so that it will not be pushed away from the atheroma 93 as to the cutting means 31 engages the atheroma. This operation continues until the cutting edge 33 engages the atheroma and begins cutting the portion of the atheroma within the cutout 14 to remove the portion which is shown as cross hatched in Figure 2C. As the cross hatched portion is removed in the cutting operation in the manner shown in Figure 2D it is collected within the cup-shaped cutting means 31 and advanced into the forward conical-shaped extremity of the housing 12. The inflated balloon 67 serves to ensure that the atheroma 93 remains in the cutout during operation of the cutting means 31.

Thereafter, the entire atherectomy device 11 can be removed from the arterial vessel as shown in Figure 2E taking with it the portion of the atheroma which has been severed. After the device has been removed from the arterial vessel, the material collected within the housing 12 is removed. In removing the atherectomy device, the guide wire can be left in place so that multiple cutting operations can be performed if necessary to remove sufficient material from the atheroma. If multiple cutting operations are to be performed on the same atheroma, the device can be removed after each cutting operation and cleaned. Additional portions of the atheroma can be removed with additional passes of the cutting means 31 until a sufficiently large passage is established to ensure that there is very little likelihood of any further possibility of occlusion of the blood vessel.

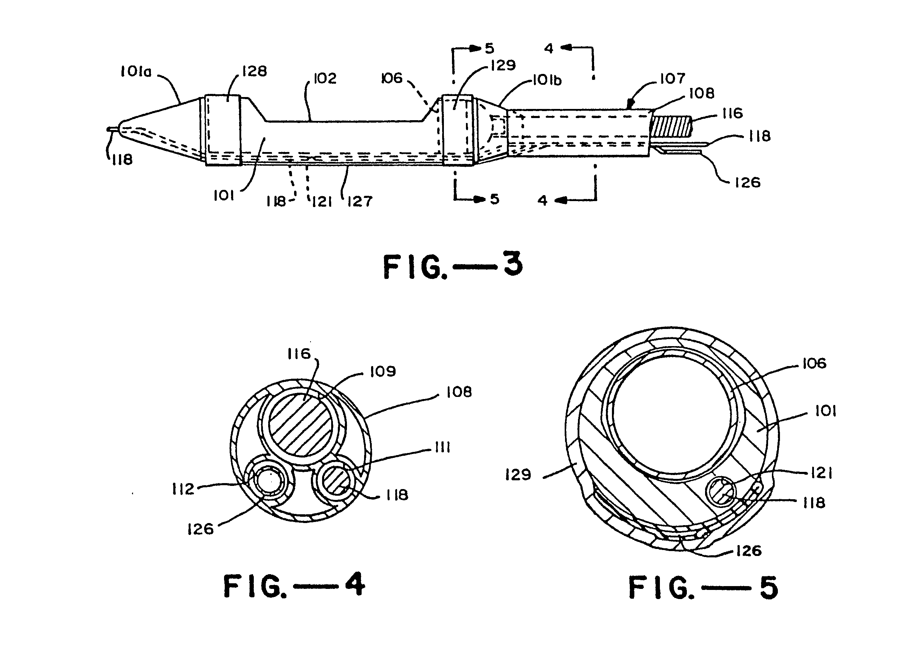

Another embodiment of the atherectomy device is shown in Figures 3 through 5. As shown therein, it also consist of a cylindrical housing 101 which is formed of plastic and which is provided with an elongate cutout 102 similarly to the cutout 14 hereinbefore described. The distal extremity of the housing is provided with a rounded portion 101a and similarly the proximal extremity of the housing 101 is provided with a rounded portion 101b. This rounded portion 101b can be formed as a separate part and bonded to the main body of the housing 101 by suitable means such as an adhesive after the atheroma cutting means or cutter 106 has been mounted within the cylindrical housing. The cutter 106 can be identical to the atheroma cutting means 31 hereinbefore described and is movable longitudinally of the housing 101.

Flexible guiding means 107 is secured to the proximal extremity of the conical portion 101b of the housing 101 and consists of a flexible plastic tube 108. The tube 108, as shown, can be formed as an extruded member which is provided with three separate lumens 109, 111, and 112.

Flexible drive means in the form of a cable 116 is provided for driving the cutter 106. The cable 116 can be hollow or solid. It is shown as being solid in Figure 4 and is disposed in the large lumen 109 provided in the tube 108. The distal extremity of the flexible cable 116 can be secured to the cutter 106 by a suitable means such as an adhesive. The flexible tube 108 can be secured to the leg 48 of the U-shaped member 47 in the same manner that the guiding cable 36 is secured thereto. Similarly, the drive cable 116 can be secured to the knob 52 in the same manner that the flexible drive means 41 is secured to the knob 52. A flexible guide wire 118 is provided which extends through the lumen 111. The lumen 111 is in communication with a passageway 121 which is provided in the plastic housing 101. The passageway 121 extends the length of the housing 101 and curves up towards the distal extremity of the same so that the passage 121 exits centrally of the housing 101. As can be seen, the guide wire 118 extends through this passageway 121 and exits through the rounded portion 101a so that it can be utilized for guiding the housing 101 in its travel through the arterial blood vessel.

A balloon tube 126 extends through the lumen 112 and is provided with an integral inflatable balloon 127 of the same type as balloon 67. The inflatable balloon 127 is secured to the housing 101 by plastic bands 128 and 129.

The proximal extremities of the guide wire 118 and the balloon tube 126 can be brought out at the proximal extremity of tube 108 adjacent the U-shaped member 47. The balloon tube 126 can be connected to an inflating device such as device 76 as shown in Figure 1B. The guide wire 118 can be manipulated in a conventional manner.

Operation of this embodiment of the atherectomy device shown in Figures 3 through 5 may now be briefly described as follows. The operation of the device is very similar to that hereinbefore described with respect to the embodiment as shown in Figures 1A and 1B. The principal difference between the two embodiments is that the guide wire 118 need not pass through the cutter 106 or through the center of the housing, as is the case, in the embodiment shown in Figures 1A and 1B. Rather, as shown, the guide wire is introduced through the housing through a passageway 121 provided in the housing. This makes it possible to insert and remove the guide wire 118 from the atherectomy device even though material may already have been cut by the cutter 106 and be disposed within the housing 101.

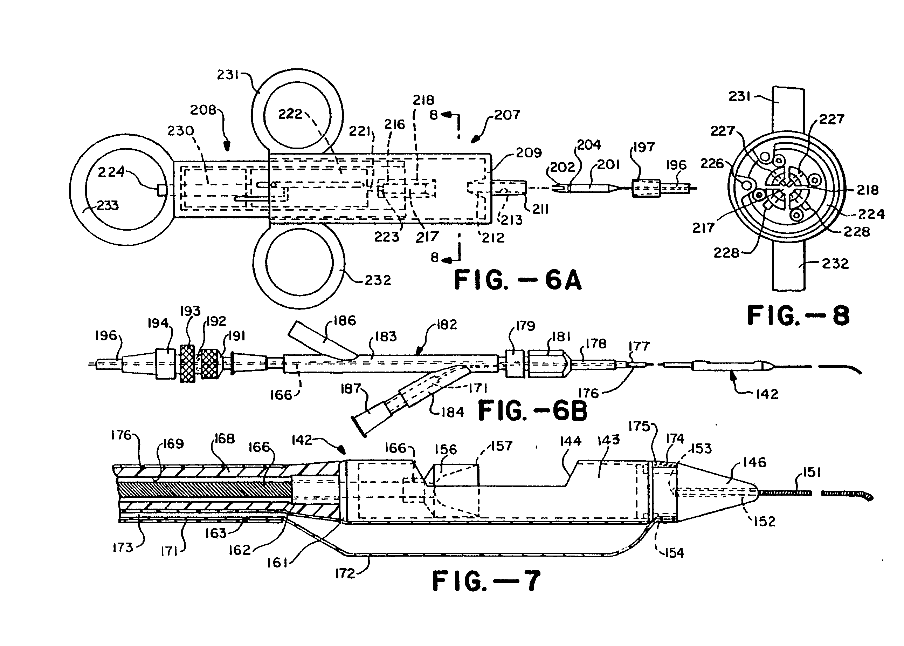

Another embodiment of the atherectomy device is shown in Figures 6A and 6B and in Figures 7 and 8. As shown therein, the atherectomy device 141 consists of a housing 142. The housing 142 is formed of a suitable material such as stainless steel and has a generally cylindrical configuration. The housing 142 consists of a cylinder 143 and is provided with a cut-out 144 extending longitudinally of the same. The cut-out 144 is shaped in a manner similar to the cut-out 14 in the embodiment of the atherectomy device shown in Figures 1A and 1B. A conical nose piece 146 is provided which is fitted into the distal extremity of the cylinder 143 by a suitable means such as an adhesive. A guide wire 151 is mounted in the nose piece 146 and extends forwardly therefrom. It has its proximal extremity positioned in a bore 152 provided in the nose piece and is soldered into the nose piece at 153 within a cylindrical recess 154 provided in the proximal extremity of the nose piece. The guide wire 151 can be of any suitable type such as a coil spring 151 formed of a radiopaque material such as tungsten.

Cutting means in the form of a cutter 156 is provided in the housing 142. The cutter 156 is similar to the cutter 31 shown in Figure 1A. It is cup shaped and is provided with a continuous circular cutting edge 157.

A tail piece 161 is mounted in the proximal extremity of the cylinder 143 and is secured therein by suitable means such as an adhesive. The tail piece 161 is provided with a rearwardly extending boss 162. The last piece 161 is provided with a bore 163 extending therethrough. Means is provided for rotating and advancing the cutter 156 and consists of a torque cable 166. The distal extremity of the torque cable 166 extends through the bore 163 and is secured to a boss 167 provided as a part of the cutter 156 by suitable means such as an adhesive. The torque cable 166 is housed in a torque tube 168 which is fitted over the proximal extremity of the tail piece 161 as shown particularly in Figure 7. The torque tube 168 can be formed of any suitable material such as plastic. A lumen 169 is formed in the torque tube through which the torque cable 166 passes and through which a radiocontrast liquid can pass as hereinafter described.

A balloon tube 171 is provided which lies adjacent to the torque tube 168. The balloon tube 171 has a balloon 172 formed integral therewith on the distal extremity of the same. The balloon tube 171 has a lumen 173 which extends through the same which is in communication with the interior of the balloon 172 so that the balloon 172 can be inflated and deflated as hereinafter described. The distal extremity of the balloon 172 is secured to the nose piece 146 in the recess 154 by a suture 174 formed of a suitable material such as Nylon (Registered Trade Mark) wrapped about the distal extremity of the balloon in the recess 154. This suture 174 is potted in a urethane compound 175 so as to provide a smooth surface and a smooth transition between the outer cylindrical surface of the cylinder 143 and the conical nose piece 146.

A third tube 176 is provided which encloses both the torque tube 168 and the balloon tube 171 and can be formed of a suitable material such as a heat shrinkable plastic so that the three tubes are tightly encased into a unitary assembly. The distal extremity of the tube 176 serves to retain the proximal extremity of the balloon 172 in tight engagement with the proximal extremity of the housing 142 so that the balloon will only be formed along the length of the housing 142 to the rear of the conical nose piece 146 and underlying the cutout 144.

In order that the atherectomy device 141 can be readily introduced into the vessels of the patient, the tube 176 can be coated with a lubricious material such as MDX supplied by Dow Corning which is essentially a silicone-based coating or alternatively, a Teflon-type coating such as DuPont Vydex. In addition, in order to provide a very smooth and still relatively hard surface, the stainless steel parts for the housing 142 can be coated with titanium nitride.

The proximal extremity of the tube 176 is secured to shrink tube sections 177 and 178 of progressively larger diameters. The tube section 178 is fitted over the distal extremity of a fitting 179 and is secured thereto by a cap 181 which is threaded onto the fitting 179. The fitting 179 is connected to a three-arm adapter 182 which is provided with a central arm 183 and two side arms 184 and 186. The balloon tube 171 extends through the three-arm adapter 182 and is connected to a fitting 187 mounted in the side arm 184. The fitting 184 is utilized for inflating and deflating the balloon. The side arm 186 is in communication with the lumen 169 provided in the torque tube 168 and can be utilized for injecting radiographic contrast fluid as hereinafter described.

The torque cable 166 also extends through the three arm adapter 182 and extends into a fitting 191 mounted on the central arm 183. The fitting 191 is provided with an O-ring through which the torque cable 166 passes and which is adapted to be pressed into engagement with the torque cable 166 to form a liquid-tight seal by the use of a thumb screw 193 threaded into the fitting 191. A fitting 194 is mounted on the thumb screw 193 and rotates with the thumb screw. A tube 196 has one end secured to the fitting 194 and has its other end secured to a fitting 197. The torque cable 166 extends through the tube 196 and through the fitting 197 and is secured to a first clutch member 201. The clutch member 201 has a generally cylindrical configuration and is provided with a pair of rearwardly extending ears 202 with a slot 203 therebetween. The clutch member 201 is provided with an annular recess 204.

Motive drive means is provided for engaging the clutch member 201 and for rotating the same and comprises a motor drive unit 206. The motor drive unit 206 consists of cylindrical members 207 and 208 with the cylindrical member 207 being an outer cylindrical member and the cylindrical member 208 being an inner cylindrical member with the inner cylindrical member telescoping within the outer cylindrical member 207 in much the same manner as a medical syringe. The cylindrical member 207 is provided with a distal wall 209 which has an outwardly extending centrally disposed tapered boss 211 and an inwardly extending tapered boss 212 through which a bore 213 is provided. The tapered boss 211 is formed so as to form a friction bit with respect to the fitting 197. The tapered boss 212 is adapted to be engaged by a second clutch member 216 which is adapted to mate with the first clutch member 201. The clutch member 216 is provided with a generally cylindrical recess 217 which is adapted to receive the first clutch member 201. The clutch member 216 is provided with four slits 218 spaced 90° apart. The second clutch member 216 is connected to an output shaft 221 of a small DC electric motor 222 by a set screw 223 of a conventional type. The motor 222 is mounted in the inner cylindrical member 208. The motor output shaft 221 rotates at a suitable speed as for example from 1,500 to 2,500 revolutions per minute. A DC power supply in the form of a cylindrical battery 223 is provided for energizing the motor 222. The battery 223 and the motor 222 are mounted in line within the cylindrical member 208 and are held in place by a sleeve 224 engaging the motor 222. The sleeve 224 is held in place by a C-clamp 226. A pair of pins 227 are mounted in the second clutch member 216 and extend into the recess 217 and are adapted to seat in the slot 203 of the first clutch member 201. An additional pair of pins 228 are provided in the clutch member 216 and are adapted to seat in the recess 204 of the clutch member 201. A hand operated switch 224 is also mounted on the proximal extremity of the cylindrical member 208 and has a spring contact which is adapted to engage one end of the battery 223 with the other end of the battery being in contact with a terminal of the motor 222. A lead 226 is provided for connecting the contact of the switch 224 to the other terminal of the motor 222.

Means is provided for grasping the motor drive unit 206 so that it can be held and operated by one hand. To this end there have been provided a pair of finger rings 231 and 232 which are mounted on the proximal extremity of the cylindrical member 207 diametrically opposite each other which are adapted to be engaged by two fingers of a hand. In addition there has been provided a circular finger member 233 which is mounted on the proximal extremity of the cylindrical member 208 and is adapted to be engaged by the thumb of the same hand which is utilized for the finger members 231 and 232. In this way, the motor drive unit 206 can be held by one hand with two fingers in the finger members 231 and 232 and the thumb in the finger member 233. The thumb can be used to operate the switch 224 to energize the motor 222.

The motive drive means is provided with means permitting the cutter 156 to be advanced and retracted while it is being rotated and consists of a guide slot 236 provided on the outer wall of the cylindrical member 208 extending longitudinally of the same for a predetermined distance. The recess 236 is adapted to be engaged by a set screw 237 mounted in the side wall of the cylindrical member 207. This permits relative longitudinal movement of the cylindrical member 208 with respect to the member 207 between the extremities of the slot or recess 236. An additional slot 241 is provided adjacent and parallel to the slot 236. The slot 241, although shorter than slot 236, extends rearwardly of the cylindrical member 208 for a distance beyond that of the slot or recess 236. A transverse slot 242 is also provided which is in communication with the slots 236 and 241.

It can be seen that by rotating the cylindrical members 207 and 208 with respect to each other, the pin 237 can be moved into either the slot 241 or the slot 236 by use of the transverse slot 242. When the pin 237 is moved into the slot 241, the cylindrical member 208 can be moved forwardly into the cylindrical member 207 so that the second clutch member 216 engages the conically shaped boss 212 to spread apart the clutch member 218 to permit the first clutch member 201 to be inserted therein or to be removed therefrom.

Let it be assumed that it is desired to insert the first clutch member 201 into the second clutch member 216. This can be accomplished merely by moving the cylindrical member 208 forwardly in the member 207 in the slot 241 so that the second clutch member 216 comes into engagement with the cylindrical boss 212 to spread apart the distal extremities of the second clutch member 216. The clutch member 201 can be then inserted into the second clutch member so that at least one of the pins 227 seats in the slot 203 and so that the pins 228 seat in the recess 204. The fitting 197 can then be pressed onto the boss 211. The cylindrical member 208 can then be retracted so that the second clutch member is no longer opened by the conical boss 212 permitting it to frictionally grip the second clutch member 201. Thereafter, the cylindrical member 208 can be rotated so that the pin 237 is moved into the slot 236. The atherectomy device is now ready for use.

The atherectomy device 141 which is shown in Figures 6A, 6B and 7 can be utilized much in the same manner as the atherectomy device hereinbefore described. However, it has been found that by providing a motorized drive, the cutting operation performed by the cutter 156 is much more predictable and permits removable of material from the vessel in a fashion as to leave surgically smooth margins in the area in which material has been removed. With the motor drive unit 206 for the atherectomy device, the cutting operation can be performed relatively rapidly. At the same time that the cutter 156 is rotated it can be advanced by hand through the desired distance. This distance is determined by the length of the slot 236. The separation of the slot 236 from the 241 ensures that the first and second clutch members will not automatically be disengaged. As explained previously, this can only be accomplished by moving the pin 237 into the slot 241.

It has been found that by driving the cutter 156 at high speeds as, for example, the 1,500 to 2,500 revolutions per minute heretofore mentioned that the cutting operation can be performed with great precision. It should be appreciated that during the time the atherectomy device 141 is being used, the cutter 156 can be moved back and forth merely by controlling the relative positions of the cylindrical members 208 and 207 with respect to each other while at the same time that the cutter is being rotated by operating the switch 224 to energize the motor 222. The desired control means can be provided for adjusting the speed of rotation of the motor. The entire procedure can be observed under an x-ray apparatus since the position of the cutter and its travel can be visually observed.

Typically, the atherectomy device can be inserted into the vessel of the patient until the cut-out 144 is in the desired position in the stenosis after which the cutter can be advanced. After the cutter has been advanced through its length of travel, the atherectomy device can be removed and the cutter emptied of the material which has been removed. The atherectomy device can again be inserted into the vessel and another cutting operation performed. If necessary, this procedure can be repeated to ensure that an adequate opening has been provided in the vessel. To ensure that a uniform opening has been provided in the vessel, the housing 142 can be rotated into different positions so that the cut-out 144 faces different areas of the stenosis so that a smooth passage is formed in the vessel in which the operation is being performed.

Typically in using the atherectomy device, a radiocontrast dye is introduced into the vessel to ascertain where the stenosis is located. After the stenosis is located, the atherectomy device can be inserted into the stenosis by the use of the guide wire 151. After it is positioned in the stenosis and the housing rotated into the desired position so that the cut-out 144 faces the narrowing in the vessel, the balloon 172 can be inflated by introducing an inflation medium through the fitting 187. Radiographic contrast fluid can be introduced through the side arm 186 so it can pass through the lumen 169 provided in the torque tube 168. The O-ring seal 192 provided prevents liquids from the vessel from leaking from the atherectomy device.

If there is a substantial narrowing in the stenosis, it will be necessary to repeat the procedure a number of times as, for example, from as many as 7 to 20 passes. Upon each successive pass the housing can be rotated by a certain number of degrees as, for example, 10 degrees. If desired, successive passes can be separated by as many as 90°.

The atherectomy device 141 can have housings of various sizes ranging from 12 French down to 4 and 5 French and possibly as low as a 3 French, even though at the smaller sizes the material removed by the device in each pass is quite small. However, even with such a small device it is believed that there are applications for the device in areas where very small diameter vessels are being surgically treated.

It is apparent from the foregoing that there has been provided an atherectomy device which is particularly efficacious in removing material from atheromas in arterial vessels. This is particularly advantageous in that it is less likely that such atheromas will reoccur after they have been removed surgically by the device of the present invention.

## Claims

1. An atherectomy device for removal of material of an atheroma in a vessel comprising a generally cylindrical housing (12) formed of a rigid material, said housing having a rounded distal end portion (26), said housing being formed with a cut-out (14) on one side of the housing, cutter means (31) disposed within the housing and movable in the housing longitudinally of the cut-out, a flexible guiding means (36) secured to the housing, a flexible drive means (41) disposed within the guiding means and secured to the cutter, and motive means secured to the drive means for rotating the cutter and permitting rotation of the cutter while the cutter is moved longitudinally of the housing; characterised in that the cut-out (14) extends longitudinally of the housing so that the remaining portion of the housing in the vicinity of the cut-out forms substantially a semi-cylinder.

2. A device as in claim 1 and further comprising an inflatable means (66) carried by the housing (12) in a region opposite the cut-out (14), a tube (69) connected to the inflatable means and having a lumen (77) therein in communication with the interior of the inflatable means and means (76) for inflating the inflatable means so that the housing can be urged in a direction towards the atheroma to retain the housing in engagement with the atheroma during the operation of the cutter means (31).

3. An atherectomy device as in claim 2 characterised in that an additional tube (78) encloses said tube (69) in communication with the inflatable means and the guiding means (36).

4. A device as claimed in any of claims 1 to 3 further comprising an adapter (182—Figure 6B) secured to the guiding means (168) and means (186) for introducing a radiographic contrast liquid into the adapter and into the guiding means.

5. A device as in any of claims 1 to 4, characterised in that said motive means includes an electric motor (222) having an output shaft (221) and clutch means (201) for connecting the output shaft of the electrical motor to the flexible drive means (166).

6. A device as in any of claims 1 to 5 and further comprising first and second cylindrical members (207, 208), the second cylindrical member (208) being slidably mounted in the first cylindrical member (207), a motor (222) carried by the second movable member, clutch means (201) connecting the output shaft (221) of the motor to the flexible drive means (166) and means (231, 232, 233) carried by the first and second cylindrical members permitting the first and second cylindrical members to be held by a single hand and permitting longitudinal movement of the second cylindrical member with respect to the first movable member.

7. A device as in claim 6 and further comprising switch means (224) carried by the second cylindrical member (208) whereby the motor (222) can be operated by the same hand that is holding the first and second cylindrical members.

8. A device as in claim 7 and further comprising means (237) carried by the second movable member (208) for causing engagement and disengagement of the clutch means and means carried by the first and second cylindrical members to prevent accidental disengagement of the clutch means during the time that the cutter is being moved longitudinally of the housing.

9. An atherectomy device as in any of claims 1 to 8, characterised in that the means on the housing for guiding the flexible member (36) is a fixed guide wire (61) secured to the distal end of the housing.

10. An atherectomy device as in any of claims 1 to 9, characterised in that the means on the housing for guiding the flexible member is an axial passage through the housing capable of receiving a guide wire.

**Patentansprüche**

1. Atherektomie-Vorrichtung zum Entfernen von Material eines Atheroms in einem Blutgefäß, mit einem im wesentlichen zylindrischen Gehäuse (12) aus formfestem Werkstoff mit einem abgerundeten distalen Endbereich (26), das auf einer Seite einen Ausschnitt (14) aufweist, mit einer in dem Gehäuse angeordneten Schneidvorrichtung (31), die in dem Gehäuse in Längsrichtung des Ausschnitts bewegbar ist, mit einem an dem Gehäuse befestigten flexiblen Führungsmittel (36), mit einem in diesem Führungsmittel angeordneten und an der Schneidvorrichtung befestigten Antriebsmittel (41) sowie mit einer mit dem Antriebsmittel verbundenen Antriebsvorrichtung zum Drehen der Schneidvorrichtung, die ein Drehen der Schneidvorrichtung ermöglicht, während diese in Längsrichtung des Gehäuses bewegt wird, dadurch gekennzeichnet, daß der Ausschnitt (14) sich in der Weise in Längsrichtung des Gehäuses erstreckt, daß der verbleibende Teil des Gehäuses im Bereich des Ausschnitts im wesentlichen einen Halbzylinder bildet.

2. Atherektomie-Vorrichtung nach Anspruch 1, gekennzeichnet, durch ein an dem Gehäuse (12) in einem dem Ausschnitt (14) entgegengesetzten Bereich angebrachtes aufblasbares Mittel (66), ein mit diesem aufblasbaren Mittel verbundenes Rohr (69) mit einem inneren Hohlraum, der mit dem Innenraum des aufblasbaren Mittels in Verbindung steht, und eine Einrichtung (76) zum Aufblasen des aufblasbaren Mittels in der Weise, daß das Gehäuse in Richtung gegen das Atherom gedrückt werden kann und während der Betätigung der Schneidvorrichtung (31) mit dem Atherom in Eingriff gehalten wird.

3. Atherektomie-Vorrichtung nach Anspruch 2, dadurch gekennzeichnet, daß ein zusätzliches Rohr (78) das genannte Rohr (69) umschließt das mit dem aufblasbaren Mittel und dem Führungsmittel (36) in Verbindung steht.

4. Atherektomie-Vorrichtung nach einem der Ansprüche 1 bis 3, gekennzeichnet durch einen an dem Führungsmittel (168) befestigten Adapter (182, Fig. 6B) und Mittel (186) zum Einführen einer Röntgenkontrastlösung in den Adapter und in das Führungsmittel.

5. Atherektomie-Vorrichtung nach Anspruch 1 bis 4, dadurch gekennzeichnet, daß die Antriebsvorrichtung einen Elektromotor (222) mit einer Ausgangswelle (221) umfaßt sowie eine Kupplungsvorrichtung (201) zum Verbinden der Ausgangswelle des Elektromotors mit dem flexiblen Atnriebsmittel (166).

6. Atherektomie-Vorrichtung nach einem der Ansprüche 1 bis 5, gekennzeichnet durch ein erstes und ein zweites zylindrisches Teil (207, 208), wobei das zweite zylindrische Teil (208) in dem ersten zylindrischen Teil gleitbar montiert ist, einen von dem zweiten Teil getragenen Motor (222), eine Kupplungsvorrichtung (201), die die Ausgangswelle (221) des Motors mit dem flexiblen Antriebsmittel (166) verbindet und von dem ersten und dem zweiten zylindrischen Teil getragene Mittel (231, 232, 233), die das Halten des ersten und des zweiten zylindrischen Teils mit nur einer Hand und eine Längsbewegung des zweiten zylindrischen Teils relativ zu dem ersten zylindrischen Teil ermöglichen.

7. Atherektomie-Vorrichtung nach Anspruch 6, gekennzeichnet durch Schaltmittel (224), die an dem zweiten zylindrischen Teil (208) so angeordnet sind, daß der Motor (222) von derselben Hand geschaltet werden kann, die das erste und das zweite zylindrische Teil hält.

8. Atherektomie-Vorrichtung nach Anspruch 7, gekennzeichnet durch von dem zweiten zylindrischen Teil (208) getragene Kupplungsbetätigungsmittel (237), die das Einrücken und Lösen der Kupplungsvorrichtung bewirken, sowie von dem ersten und dem zweiten zylindrischen Teil getragene Mittel, die ein versehentliches Lösen der Kupplungsvorrichtung während der Zeit, in der die Schneidvorrichtung in Längsrichtung des Gehäuses bewegt wird, verhindern.

9. Atherektmoie-Vorrichtung nach einem der Ansprüche 1 bis 8, dadurch gekennzeichnet, daß das Mittel an dem Gehäuse zum Führen des flexiblen Teils (36) ein an dem distalen Endbereich des Gehäuses festgelegter fester Führungsdraht (61) ist.

10. Atherektomie-Vorrichtung nach einem der Ansprüche 1 bis 9, dadurch gekennzeichnet, daß das Mittel an dem Gehäuse Führen des flexiblen Teils ein axialer Durchgang durch das Gehäuse ist, der einen Führungsdraht aufnehmen kann.

**Revendications**

1. Dispositif d'athérectomie pour retirer de la matière d'un athérome dans un vaisseau, comprenant un boîtier (12) généralement cylindrique constitué par une matière rigide, ledit boîtier ayant une partie d'extrémité distale (26) arondie, ledit boîtier présentant une découpure (14) sur un côté du boîtier, une lame (31) disposée à l'intérieur du boîtier et mobile dans le boîtier le long de la découpure, un moyen de guidage souple (36) fixé au boîtier, un moyen d'entraînement souple (41) disposé à l'intérieur du moyen de guidage et fixé à la lame, et un moyen moteur fixé au moyen d'entraînement pour faire tourner la lame et permettant la rotation de la lame tandis que la lame est déplacée le long du boîtier, caractérisé en ce que la découpure (14) s'étend le long du boîtier de sorte que la partie restante du boîtier au voisinage de la découpure forme sensiblement un demi-cylindre.

2. Dispositif selon la revendication 1 et comprenant en outre un moyen gonflable (66) porté par le boîtier (12) dans une région opposée à la découpure (14), un tube (69) relié au moyen gonflable et présentant une ouverture (77) à l'intérieur en communication avec l'intérieur du moyen gonflable et un moyen (76) pour gonfler le moyen gonflable de sorte que le boîtier peut être poussé vers l'athérome pour maintenir le boîtier en contact avec l'athérome au cours de l'actionnement de la lame (31).

3. Dispositif d'athérectomie selon la revendication 2, caractérisé en ce qu'un tube supplémentaire (78) entoure ledit tube (69) en communication avec le moyen gonflable et le moyen de guidage (36).

4. Dispositif selon l'une des revendications 1 à 3, comprenant en outre un adaptateur (182—figure 6B) fixé au moyen de guidage (168) et un moyen (186) pour introduire un liquide de contraste radiographique dans l'adaptateur et dans le moyen de guidage.

5. Dispositif selon l'une des revendications 1 à 4, caractérisé en ce que ledit moyen moteur comprend un moteur électrique (222) ayant un arbre de sortie (221) et un embrayage (201) pour relier l'arbre de sortie du moteur électrique au moyen d'entraînement souple (166).

6. Dispositif selon l'une des revendications 1 à 5 et comprenant en outre des premier et second éléments cylindriques (207, 208), le second élément cylindrique (208) étant monté dans le premier élément cylindrique (207) de façon à pouvoir glisser, un moteur (222) porté par le second élément mobile, un embrayage (201) reliant l'arbre de sortie (221) du moteur au moyen d'entraînement souple (166) et des moyens (231, 232, 233) portés par les premier et second éléments cylindriques permettant aux premier et second éléments cylindriques d'être maintenus d'une seule main et permettant un mouvement longitudinal du second élément cylindrique par rapport au premier élément mobile.

7. Dispositif selon la revendication 6 et comprenant en outre un interrupteur (224) porté par le seconde élément cylindrique (208) et par lequel le moteur (222) peut être actionné par la même main que celle qui tient les premier et second éléments cylindriques.

8. Dispositif selon la revendication 7 et comprenant en outre un moyen (237) porté par le second élément mobile (208) pour provoquer l'engrènement et le désengrènement de l'embrayage et des moyens portés par les premier et second éléments cylindriques pour empêcher un désengrènement accidentel de l'embrayage au cours du déplacement de la lame le long du boîtier.

9. Dispositif d'athérectomie selon l'une des revendications 1 à 8, caractérisé en ce que le moyen sur le boîtier pour guider l'élément souple (36) est un fil de guidage fixe (61) fixé à l'extrémité distale du boîtier.

10. Dispositif d'athérectomie selon l'une des revendications 1 à 9, caractérisé en ce que le moyen sur le boîtier pour guider l'élément souple est un passage axial dans le boîtier pouvant recevoir un fil de guidage.

FIG.—IA

FIG.—IB

EP 0 163 502 B1

FIG.—2A

FIG.—2B

FIG.—2C

FIG.—2D

FIG.—2E

FIG.—3

FIG.—4

FIG.—5

EP 0 163 502 B1

FIG.—8

FIG.—6A

FIG.—6B

FIG.—7